(19) **Europäisches Patentamt**
**European Patent Office**
**Office européen des brevets**

(11) **EP 3 851 544 A1**

(12) **EUROPEAN PATENT APPLICATION**
published in accordance with Art. 153(4) EPC

(43) Date of publication:
**21.07.2021 Bulletin 2021/29**

(21) Application number: **19859900.3**

(22) Date of filing: **09.09.2019**

(51) Int Cl.:
*C12Q 1/6888* (2018.01)　　*C12Q 1/6886* (2018.01)

(86) International application number:
**PCT/CN2019/104946**

(87) International publication number:
**WO 2020/052520 (19.03.2020 Gazette 2020/12)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME
KH MA MD TN**

(30) Priority: **10.09.2018　CN 201811052794**

(71) Applicant: **Lisen Imprinting Diagnostics Wuxi Co.,
Ltd**
**Wuxi City, Jiangsu 214000 (CN)**

(72) Inventors:
• **CHENG, Tong**
**Wuxi, Jiangsu 214000 (CN)**
• **ZHOU, Ning**
**Wuxi, Jiangsu 214000 (CN)**

(74) Representative: **Grund, Martin et al
Grund Intellectual Property Group
Patentanwalt und Solicitor PartG mbB
Postfach 44 05 16
80754 München (DE)**

(54) **HIERARCHICAL MODEL FOR DETECTING BENIGN AND MALIGNANT DEGREE OF SKIN TUMORS AND APPLICATION THEREOF**

(57) Disclosed are a grading model for detecting the benign and malignant degree of skin tumors and an application thereof. The model grades changes of imprinted genes in tumors by calculating the expression of the loss of imprinting of the imprinted genes, the expression of the copy number variation of the imprinted genes and the total expression of the imprinted genes. The detection model and device intuitively express the presentation of the loss of imprinting on tissue and cell samples of patients with skin tumors, detect the changes of imprinted genes by means of in-situ marking objectively, intuitively and accurately in an early phase, provide a quantitative model, and make a great contribution to the diagnosis of skin tumors.

Fig. 1

EP 3 851 544 A1

**Description**

**Technical Field**

The disclosure relates to the field of biotechnology such as the field of gene diagnosis, in particular to a grading model for detecting the benign and malignant degree of skin tumors and an application thereof, and a grading model for detecting the benign and malignant degree of skin tumors by means of a group of imprinted genes and a device comprising the same.

**Description of Related Art**

There are many types of skin cancers such as malignant melanoma, basal cell carcinoma, squamous cell carcinoma, eczematoid carcinoma and fibrosarcoma, all these skin cancers, except the malignant melanoma, are very common and less malignant and are not included in cancer statistical data in many countries. As estimated by the World Health Organization, there are about over 3,000,000 new cases every year. While, the malignant melanoma is highly malignant and quite likely metastasize to the whole body. According to the statistics from the World Health Organization, the malignant melanoma has an occurrence of 232,000 new cases in 2012, among which there are 55,000 death cases. Most melanoma patients are white people with a little skin melanin. The fall of the ozone level in the atmosphere gradually weakens the ultraviolet filter effect, which in turn results in a continuous rise of the morbidity of melanoma. In-situ melanoma in the early phase can be easily excised by surgery. However, due to the difficulty in pathological diagnosis of melanoma that the in-situ melanoma cannot be easily distinguished from pigmented naevi in form, the melanoma has already metastasized when some melanoma patients are confirmed, which increases the complexity of treatment and the suffering of patients. Thus, it is necessary to develop a melanoma early-detection technique with higher accuracy to save the life of a great number of patients and greatly reduce the social medical expense.

Traditional pathology determines the benignity or malignancy of cells based on the size, morphology and invasion of the cells and the relation with cell tissues around, so it has great limitations in finding early changes of cells (cancers). Hence, cancer diagnosis methods on the cell molecular level have become a research focus once. With the deeper study in the molecular biology field, more and more molecular detection techniques have been used for cancer diagnosis.

Cancers are generated due to uncontrollable cell growth/division caused by epigenetic alterations and genovariation accumulated over time. Traditional pathology determines the benignity or malignancy of skin tumors according to the size, morphology and structural variations of cells and tissues. With the development and deepening of molecular biology, more and more molecular detection techniques have been used for detecting skin cancers. From the perspective of the development process of cancers, changes at the molecular level (epigenetics and genetics) are far earlier than variations in cell morphology and tissue structure. So, molecular biological detection is more sensitive to cancer detection in an early phase.

For all the above reasons, a novel detection system and model are needed for diagnosis of skin cancers at present to analyze the changes of molecular markers of skin cancers at the cellular level based on biopsy samples of patients so as to provide more accurate pre-diagnosis and diagnosis information.

**Brief Summary of the Invention**

The objective of the disclosure is to provide an imprinted gene grading model, a diagnosis method and an application thereof.

To fulfill the above objective, the application adopts the following technical solutions:

The disclosure provides an imprinted gene grading model for skin tumors. The model grades expression states of imprinted genes by calculating changes of the expression of the loss of imprinting of the imprinted genes, the expression of the copy number variation of the imprinted genes and the total expression of the imprinted genes in skin tumors;
Wherein, the imprinted genes are any one or the combination of at least two of Z1, Z8, Z11 and Z16, the imprinted gene Z1 is Gnas, the imprinted gene Z8 is Dcn, the imprinted gene Z11 is Grb10, and the imprinted gene Z16 is Snrpn/Snurf.

The loss of imprinting means that alleles originally in a silencing state in the imprinted genes are activated (demethylated), is the most common and earliest epigenetic alteration in cancers, and can be used as a pathological marker. Relatively speaking, the proportion of the loss of imprinting in healthy cell detection is extremely low. Chinese

expressions "印记基因" and "印迹基因" of the imprinted gene have the same conception and meaning and can be replaced with each other.

The inventor finds that by calculating the expression of the loss of imprinting and the expression of the copy number variation of any one of the imprinted genes Z1, Z8, Z11 and Z16 in skin tumors, the diagnosis sensitivity to skin cancers can reach over 77.8%.

In one embodiment, if only one imprinted gene is preliminarily detected, any one of the imprinted genes Z1, Z8, Z11 and Z16 can be detected.

In one embodiment, if only one imprinted gene is preliminarily detected, any one of the imprinted genes Z8, Z1 and Z11 can be detected.

In one embodiment, if only one imprinted gene is preliminarily detected, Z1 or Z8 can be detected.

The inventor finds that if only the imprinted gene Z1 is detected, the diagnosis sensitivity to skin cancers can reach 81.8%, that if only the imprinted gene Z8 is detected, the diagnosis sensitivity to skin cancers can reach 95.5%, that if only the imprinted gene Z11 is detected, the diagnosis sensitivity to skin cancers can reach 79.5%, and that if only the imprinted gene Z16 is detected, the diagnosis sensitivity to skin cancers can reach 77.8%.

In one embodiment, if the combination of two imprinted gene is detected, the combination includes any two of Z1, Z8, Z11 and Z16, and is preferably, the combination of Z1 and Z8 or the combination of Z8 and Z11.

The inventor finds that by calculating the expression of the loss of imprinting and the expression of the copy number variation of two or more imprinted genes, the sensitivity can be further improved; when the combination of two imprinted genes is detected, the diagnosis sensitivity to skin cancers can reach over 90.0%; when the combination of Z1 and Z8 is detected, the diagnosis sensitivity to skin cancers can reach 99.0%; and when the combination of Z8 and Z11 is detected, the diagnosis sensitivity to skin cancers can reach 97.7%.

In one embodiment, the imprinted genes further include any one or the combination of at least two of Z6, Z10 and Z13, wherein the imprinted gene Z6 is Plagl1, the imprinted gene Z10 is Gatm, and the imprinted gene Z13 is Sgce.

The inventor finds that the detection accuracy can be improved by adding the genes Z6, Z10 and Z13 for combined diagnosis based on the genes Z1, Z8, Z11 and Z16, false positives can be further avoided by using probes for auxiliary diagnosis, the detection accuracy can be further improved, and thus, all skin tumor samples can be accurately graded and determined.

In one embodiment, an imprinted gene calculation method of the model comprises: calculating a combination of imprinted genes, wherein the imprinted genes are combinations of genes Z1, Z6, Z8, Z10, Z11, Z13 and Z16.

The loss of imprinting refers to the existence of two red/brown markers in the cell nucleus after a cell is stained with hematoxylin. The copy number variation of imprinted genes refers to the existence of more than two red/brown markers in the cell nucleus after a cell is stained with hematoxylin and is a phenomenon that a gene is represented as a triploid or an even higher polyploidy due to abnormal gene duplication of cancer cells.

The markers generated after hematoxylin staining are, but not limited to, red or brown, and staining markers in other colors can also be used for calculating the total expression of the imprinted genes, the expression of the loss of imprinting of the imprinted genes, and the expression of the copy number variation of the imprinted genes.

In one embodiment, formulas for calculating the total expression of the imprinted genes, the expression of the loss of imprinting of the imprinted genes, and the expression of the copy number variation of the imprinted genes are as follows:

$$\text{Total expression} = (b+c+d) / (a+b+c+d) \times 100\%;$$

$$\text{Total expression of normal imprinted genes} = b / (b+c+d) \times 100\%;$$

$$\text{Expression of the loss of imprinting (LOI)} = c / (b+c+d) \times 100\%;$$

$$\text{Gene expression of the copy number variation (CNV)} = d / (b+c+d) \times 100\%;$$

Wherein, a is a cell nucleus in which no marker exists and no imprinted gene is expressed after a cell is stained with hematoxylin; b is a cell nucleus in which one red/brown marker and an imprinted gene exist after a cell is stained with hematoxylin; c is a cell nucleus subjected to the loss of imprinting due to the existence two red/brown markers after a cell is stained with hematoxylin; and d is a cell nucleus subjected to the copy number variation due to the existence of more than two red/brown markers after a cell is stained with hematoxylin.

In one embodiment, the markers generated after hematoxylin staining are, but not limited to, red or brown, and staining markers in other colors can also be used for calculating the expression of the loss of imprinting of the imprinted genes, the expression of the copy number variation of the imprinted genes and the total expression of the imprinted genes.

In one embodiment, the existence, loss of imprinting or copy number variation of imprinted genes in each cell nucleus is determined under a 40x or 60x microscope by means of in-situ hybridization of probes and signal amplification based on nuclear staining with Hemotoxy, and the benign and malignant degree of a tumor sample is determined by calculating the expression of the loss of imprinting, the expression of the copy number variation and the total expression of the imprinted genes; and because a section is only 10μm, if about 20% of the cell nucleus seen under the microscope is incomplete, it indicates that a false negative may exist.

In one embodiment, the expression of the loss of imprinting of the imprinted genes, the expression of the copy number variation of the imprinted genes and the total expression of the imprinted genes are classified to five grades.

In one embodiment, in the most positive area of sample expression of each probe, the expression of the loss of imprinting, the expression of the copy number variation and the total expression of the seven imprinted genes Z1, Z6, Z8, Z10, Z11, Z13 and Z16 in at least 1200 cells are classified to five grades.

The five grades of the expression of the loss of imprinting, the expression of the copy number variation and the total expression of the imprinted genes Z1 and Z16 are:

Grade 0: any one or the combination of at least two of the case where the expression of the loss of imprinting of the imprinted genes Z1 and Z16 is less than 15%, the case where the expression of the copy number variation of the imprinted genes Z1 and Z16 is less than 1.5%, and the case where the total expression of the imprinted genes Z1 and Z16 is less than 25%;

Grade I: any one or the combination of at least two of the case where the expression of the loss of imprinting of the imprinted genes Z1 and Z16 is 15-20%, the case where the expression of the copy number variation of the imprinted genes Z1 and Z16 is 1.5-2.5%, and the case where the total expression of the imprinted genes Z1 and Z16 is 25-35%;

Grade II: any one or the combination of at least two of the case where the expression of the loss of imprinting of the imprinted genes Z1 and Z16 is 20-23%, the case where the expression of the copy number variation of the imprinted genes Z1 and Z16 is 2.5-3.5%, and the case where the total expression of the imprinted genes Z1 and Z16 is 35-45%;

Grade III: any one or the combination of at least two of the case where the expression of the loss of imprinting of the imprinted genes Z1 and Z16 is 23-27%, the case where the expression of the copy number variation of the imprinted genes Z1 and Z16 is 3.5-5%, and the case where the total expression of the imprinted genes Z1 and Z16 is 45-55%;

Grade IV: any one or the combination of at least two of the case where the expression of the loss of imprinting of the imprinted genes Z1 and Z16 is greater than 27%, the case where the expression of the copy number variation of the imprinted genes Z1 and Z16 is greater than 5%, and the case where the total expression of the imprinted genes Z1 and Z16 is greater than 55%;

The expression of the loss of imprinting, the expression of the copy number variation and the total expression of the imprinted genes Z1 and Z16 are mutually independent.

In one embodiment, the five grades of the expression of the loss of imprinting, the expression of the copy number variation and the total expression of the imprinted gene Z6 are:

Grade 0: any one or the combination of at least two of the case where the expression of the loss of imprinting of the imprinted gene Z6 is less than 10%, the case where the expression of the copy number variation of the imprinted gene Z6 is less than 1.5%, and the case where the total expression of the imprinted gene Z6 is less than 20%;

Grade I: any one or the combination of at least two of the case where the expression of the loss of imprinting of the imprinted gene Z6 is 10-15%, the case where the expression of the copy number variation of the imprinted gene Z6 is 1.5-2.5%, and the case where the total expression of the imprinted gene Z6 is 20-30%;

Grade II: any one or the combination of at least two of the case where the expression of the loss of imprinting of the imprinted gene Z6 is 15-23%, the case where the expression of the copy number variation of the imprinted gene Z6 is 2.5-3.5%, and the case where the total expression of the imprinted gene Z6 is 30-40%;

Grade III: any one or the combination of at least two of the case where the expression of the loss of imprinting of the imprinted gene Z6 is 23-27%, the case where the expression of the copy number variation of the imprinted gene Z6 is 3.5-5%, and the case where the total expression of the imprinted gene Z6 is 40-50%;

Grade IV: any one or the combination of at least two of the case where the expression of the loss of imprinting of the imprinted gene Z6 is greater than 27%, the case where the expression of the copy number variation of the imprinted gene Z6 is greater than 5%, and the case where the total expression of the imprinted gene Z6 is greater than 50%.

The five grades of the expression of the loss of imprinting, the expression of the copy number variation and the total expression of the imprinted gene Z8 are:

Grade 0: any one or the combination of at least two of the case where the expression of the loss of imprinting of the imprinted gene Z8 is less than 16%, the case where the expression of the copy number variation of the imprinted gene Z8 is less than 5%, and the case where the total expression of the imprinted gene Z8 is less than 10%;

Grade I: any one or the combination of at least two of the case where the expression of the loss of imprinting of the imprinted gene Z8 is 16-20%, the case where the expression of the copy number variation of the imprinted gene Z8 is 5-10%, and the case where the total expression of the imprinted gene Z8 is 10-20%;

Grade II: any one or the combination of at least two of the case where the expression of the loss of imprinting of the imprinted gene Z8 is 20-30%, the case where the expression of the copy number variation of the imprinted gene Z8 is 10-20%, and the case where the total expression of the imprinted gene Z8 is 20-30%;

Grade III: any one or the combination of at least two of the case where the expression of the loss of imprinting of the imprinted gene Z8 is 30-40%, the case where the expression of the copy number variation of the imprinted gene Z8 is 20-30%, and the case where the total expression of the imprinted gene Z8 is 30-40%;

Grade IV: any one or the combination of at least two of the case where the expression of the loss of imprinting of the imprinted gene Z8 is greater than 40%, the case where the expression of the copy number variation of the imprinted gene Z8 greater than 30%, and the case where the total expression of the imprinted gene Z8 is greater than 40%.

The five grades of the expression of the loss of imprinting, the expression of the copy number variation and the total expression of the imprinted genes Z10, Z11 and Z13 are:

Grade 0: any one or the combination of at least two of the case where the expression of the loss of imprinting of the imprinted genes Z10, Z11 and Z13 is less than 15%, the case where the expression of the copy number variation of the imprinted genes Z10, Z11 and Z13 is less than 1.5%, and the case where the total expression of the imprinted genes Z10, Z11 and Z13 is less than 20%;

Grade I: any one or the combination of at least two of the case where the expression of the loss of imprinting of the imprinted genes Z10, Z11 and Z13 is 15-20%, the case where the expression of the copy number variation of the imprinted genes Z10, Z11 and Z13 is 1.5-2.5%, and the case where the total expression of the imprinted genes Z10, Z11 and Z13 is 20-30%;

Grade II: any one or the combination of at least two of the case where the expression of the loss of imprinting of the imprinted genes Z10, Z11 and Z13 is 20-23%, the case where the expression of the copy number variation of the imprinted genes Z10, Z11 and Z13 is 2.5-3.5%, and the case where the total expression of the imprinted genes Z10, Z11 and Z13 is 30-40%;

Grade III: any one or the combination of at least two of the case where the expression of the loss of imprinting of the imprinted genes Z10, Z11 and Z13 is 23-27%, the case where the expression of the copy number variation of the imprinted genes Z10, Z11 and Z13 is 3.5-5%, and the case where the total expression of the imprinted genes Z10, Z11 and Z13 is 40-50%;

Grade IV: any one or the combination of at least two of the case where the expression of the loss of imprinting of the imprinted genes Z10, Z11 and Z13 is greater than 27%, the case where the expression of the copy number variation of the imprinted genes Z10, Z11 and Z13 is greater than 5%, and the case where the total expression of the imprinted genes Z10, Z11 and Z13 is greater than 50%;

The expression of the loss of imprinting, the expression of the copy number variation and the total expression of the imprinted genes Z10, Z11 and Z13 are mutually independent.

In one embodiment, the disclosure further provides a device for detecting the benign and malignant degree of skin tumors. The device adopts the model mentioned above and comprises:

(1) A sampling unit for acquiring to-be-detected samples;
(2) A probe design unit for designing specific primers according to an imprinted gene sequence;
(3) A detection unit for carrying out in-situ hybridization on probes designed in Step (2) and the to-be-detected samples; and
(4) An analysis unit for analyzing expressions of imprinted genes by means of microscope imaging;

Wherein, the analysis unit calculates the expression of the loss of imprinting, the expression of the copy number variation and the total expression of the imprinted genes, and then, the benign and malignant degree of skin tumors is determined through the model according to the grade of the expression of the loss of imprinting, the expression of the copy number variation and the total expression of the imprinted genes.

The loss of imprinting refers to the existence of two red/brown markers in the cell nucleus after a cell is stained with hematoxylin. The copy number variation of imprinted genes refers to the existence of more than two red/brown markers in the cell nucleus after a cell is stained with hematoxylin and is a phenomenon that a gene is represented as a triploid or an even higher polyploidy due to abnormal gene duplication of cancer cells.

The markers generated after hematoxylin staining are, but not limited to, red or brown, and staining markers in other colors can also be used for calculating the total expression of the imprinted genes, the expression of the loss of imprinting of the imprinted genes, and the expression of the copy number variation of the imprinted genes.

A detection system determines the benign the malignant degree of skin tumors by intuitively observing changes of imprinted genes of skin tumors in the early phase at the cell and tissue level to provide a most favorable treatment opportunity for patients with skin tumors.

In one embodiment, the disclosure further provides a method for detecting the benign and malignant degree of skin tumors. The method adopts the model or the device mentioned above, and comprises:

(1) Acquiring to-be-detected samples;
(2) Designing specific primers according to an imprinted gene sequence;
(3) Carrying out in-situ hybridization on probes designed in Step (2) and the to-be-detected samples; and
(4) Analyzing expressions of imprinted genes by means of microscope imaging to diagnose the benign and malignant degree of skin tumors;

Wherein, the analysis unit calculates the expression of the loss of imprinting, the expression of the copy number variation and the total expression of the imprinted genes, and then, the benign and malignant degree of skin tumors is determined through the model according to the grade of the expression of the loss of imprinting, the expression of the copy number variation and the total expression of the imprinted genes.

In one embodiment, the to-be-tested samples acquired in Step (1) are tissues and/or cells of humans.

Any to-be-tested samples with the RNA being fixed in time are feasible, and can be selected by those skilled in the art as needed, and the invention has no specific limitation in this aspect. The to-be-tested samples are any one or the combination of at least two of paraffin sections of tissues and skin needle biopsy samples.

The specific operating steps of the paraffin sections of the tissues are: acquiring a human tumor tissue sample, fixing the human tumor tissue sample in time with 10% neutral formalin, carrying out paraffin embedding, cutting the sample to a thickness of $10\mu m$, and preparing a tissue section through a slide with positive charges. Because the section is only $10\mu m$ thick, the cell nucleus is partially incomplete when seen under a microscope, and a loss of imprinting with partial false negative will occur.

The specific operating steps of the skin needle biopsy samples are: acquiring a human cell through puncture, and fixing the human cell in time with 10% neutral formalin.

Needle biopsy does little harm to patients, has a simple sampling process, and can be positioned according to the blood circulation characteristic, and thus, needle biopsy samples have special advantages when used as experimental samples.

In one embodiment, the to-be-tested samples skin needle biopsy samples.

In one embodiment, the imprinted genes are Z1, Z6, Z8, Z10, Z11, Z13 and Z16, wherein the imprinted gene Z1 is Gnas, the imprinted gene Z6 is Plagl1, the imprinted gene Z8 is Dcn, the imprinted gene Z10 is Gatm, the imprinted gene Z11 is Grb10, the imprinted gene Z13 is Sgce, and the imprinted gene Z16 is Snrpn/Snurf.

The degree of the expressions of the imprinted genes Z1(Gnas), Z6(Plagl1), Z8(Dcn), Z10(Gatm), Z11(Grb10), Z13(Sgce) and Z16(Snrpn/Snurf) varies in normal tumor cell tissues, and when a malignant lesion happens, both the expression and the imprinting state will change obviously.

The probes are designed according to the imprinted genes Z1, Z6, Z8, Z10, Z11, Z13 and Z16, namely Gnas, Plagl1, Dcn, Gatm, Grb10, Sgce and Snrpn/Snurf. Specifically, a fragment is selected in the intron of each gene to be used as a probe. More specifically, the probes are designed by Advanced Cell Diagnostics.

In one embodiment, the in-situ hybridization is RNAscope in-situ hybridization.

In one embodiment, the RNAscope in-situ hybridization uses a single-channel or multi-channel chromogenic kit, or a single-channel or multi-channel fluorescent kit, and is preferably a single-channel red/brown chromogenic kit or a multi-channel fluorescent kit.

The multi-channel chromogenic or fluorescent kit is a chromogenic or fluorescent kit with two or more channels, and two-channel chromogenic kit or the multi-channel fluorescent kit may adopt the combined expression of two imprinted gene probes or imprinted genes and other genes or even the comprehensive expression of multiple imprinted genes and non-imprinted genes.

In one embodiment, formulas for calculating the total expression of the imprinted genes, the expression of the loss of imprinting of the imprinted genes, and the expression of the copy number variation of the imprinted genes are

as follows:

$$\text{Total expression} = (b+c+d) / (a+b+c+d) \times 100\%;$$

$$\text{Total expression of normal imprinted genes} = b / (b+c+d) \times 100\%;$$

$$\text{Expression of the loss of imprinting (LOI)} = c / (b+c+d) \times 100\%;$$

$$\text{Gene expression of the copy number variation (CNV)} = d / (b+c+d) \times 100\%;$$

Wherein, a is a cell nucleus in which no marker exists and no imprinted gene is expressed after a cell is stained with hematoxylin; b is a cell nucleus in which one red/brown marker and an imprinted gene exist after a cell is stained with hematoxylin; c is a cell nucleus subjected to the loss of imprinting due to the existence two red/brown markers after a cell is stained with hematoxylin; and d is a cell nucleus subjected to the copy number variation due to the existence of more than two red/brown markers after a cell is stained with hematoxylin.

The markers generated after hematoxylin staining are, but not limited to, red or brown, and staining markers in other colors can also be used for calculating the total expression of the imprinted genes, the expression of the loss of imprinting of the imprinted genes and the expression of the copy number variation of the imprinted genes.

The existence, loss of imprinting or copy number variation of imprinted genes in each cell nucleus is determined under a 40x or 60x microscope by means of in-situ hybridization of probes and signal amplification based on nuclear staining with Hemotoxy, and the benign and malignant degree of a tumor sample is determined by calculating the expression of the loss of imprinting, the expression of the copy number variation and the total expression of the imprinted genes; and because a section is only 10μm, if about 20% of the cell nucleus seen under the microscope is incomplete, it indicates that a false negative may exist.

In one embodiment, the expression of the loss of imprinting of the imprinted genes, the expression of the copy number variation of the imprinted genes and the total expression of the imprinted genes are classified to five grades.

The five grades are obtained by classifying the expression of the loss of imprinting, the expression of the copy number variation and the total expression of the seven imprinted genes Z1, Z6, Z8, Z10, Z11, Z13 and Z16 in at least 1200 cells, in the most positive area of sample expression of each probe.

The five grades of the expression of the loss of imprinting, the expression of the copy number variation and the total expression of the imprinted genes Z1 and Z16 are:

Grade 0: any one or the combination of at least two of the case where the expression of the loss of imprinting of the imprinted genes Z1 and Z16 is less than 15%, the case where the expression of the copy number variation of the imprinted genes Z1 and Z16 is less than 1.5%, and the case where the total expression of the imprinted genes Z1 and Z16 is less than 25%;

Grade I: any one or the combination of at least two of the case where the expression of the loss of imprinting of the imprinted genes Z1 and Z16 is 15-20%, the case where the expression of the copy number variation of the imprinted genes Z1 and Z16 is 1.5-2.5%, and the case where the total expression of the imprinted genes Z1 and Z16 is 25-35%;

Grade II: any one or the combination of at least two of the case where the expression of the loss of imprinting of the imprinted genes Z1 and Z16 is 20-23%, the case where the expression of the copy number variation of the imprinted genes Z1 and Z16 is 2.5-3.5%, and the case where the total expression of the imprinted genes Z1 and Z16 is 35-45%;

Grade III: any one or the combination of at least two of the case where the expression of the loss of imprinting of the imprinted genes Z1 and Z16 is 23-27%, the case where the expression of the copy number variation of the imprinted genes Z1 and Z16 is 3.5-5%, and the case where the total expression of the imprinted genes Z1 and Z16 is 45-55%;

Grade IV: any one or the combination of at least two of the case where the expression of the loss of imprinting of the imprinted genes Z1 and Z16 is greater than 27%, the case where the expression of the copy number variation of the imprinted genes Z1 and Z16 is greater than 5%, and the case where the total expression of the imprinted genes Z1 and Z16 is greater than 55%;

The expression of the loss of imprinting, the expression of the copy number variation and the total expression of the imprinted genes Z1 and Z16 are mutually independent.

The five grades of the expression of the loss of imprinting, the expression of the copy number variation and the total expression of the imprinted gene Z6 are:

Grade 0: any one or the combination of at least two of the case where the expression of the loss of imprinting of the imprinted gene Z6 is less than 10%, the case where the expression of the copy number variation of the imprinted gene Z6 is less than 1.5%, and the case where the total expression of the imprinted gene Z6 is less than 20%;

Grade I: any one or the combination of at least two of the case where the expression of the loss of imprinting of the imprinted gene Z6 is 10-15%, the case where the expression of the copy number variation of the imprinted gene Z6 is 1.5-2.5%, and the case where the total expression of the imprinted gene Z6 is 20-30%;

Grade II: any one or the combination of at least two of the case where the expression of the loss of imprinting of the imprinted gene Z6 is 15-23%, the case where the expression of the copy number variation of the imprinted gene Z6 is 2.5-3.5%, and the case where the total expression of the imprinted gene Z6 is 30-40%;

Grade III: any one or the combination of at least two of the case where the expression of the loss of imprinting of the imprinted gene Z6 is 23-27%, the case where the expression of the copy number variation of the imprinted gene Z6 is 3.5-5%, and the case where the total expression of the imprinted gene Z6 is 40-50%;

Grade IV: any one or the combination of at least two of the case where the expression of the loss of imprinting of the imprinted gene Z6 is greater than 27%, the case where the expression of the copy number variation of the imprinted gene Z6 is greater than 5%, and the case where the total expression of the imprinted gene Z6 is greater than 50%.

The five grades of the expression of the loss of imprinting, the expression of the copy number variation and the total expression of the imprinted gene Z8 are:

Grade 0: any one or the combination of at least two of the case where the expression of the loss of imprinting of the imprinted gene Z8 is less than 16%, the case where the expression of the copy number variation of the imprinted gene Z8 is less than 5%, and the case where the total expression of the imprinted gene Z8 is less than 10%;

Grade I: any one or the combination of at least two of the case where the expression of the loss of imprinting of the imprinted gene Z8 is 16-20%, the case where the expression of the copy number variation of the imprinted gene Z8 is 5-10%, and the case where the total expression of the imprinted gene Z8 is 10-20%;

Grade II: any one or the combination of at least two of the case where the expression of the loss of imprinting of the imprinted gene Z8 is 20-30%, the case where the expression of the copy number variation of the imprinted gene Z8 is 10-20%, and the case where the total expression of the imprinted gene Z8 is 20-30%;

Grade III: any one or the combination of at least two of the case where the expression of the loss of imprinting of the imprinted gene Z8 is 30-40%, the case where the expression of the copy number variation of the imprinted gene Z8 is 20-30%, and the case where the total expression of the imprinted gene Z8 is 30-40%;

Grade IV: any one or the combination of at least two of the case where the expression of the loss of imprinting of the imprinted gene Z8 is greater than 40%, the case where the expression of the copy number variation of the imprinted gene Z8 is greater than 30%, and the case where the total expression of the imprinted gene Z8 is greater than 40%.

Preferably, the five grades of the expression of the loss of imprinting, the expression of the copy number variation and the total expression of the imprinted genes Z10, Z11 and Z13 are:

Grade 0: any one or the combination of at least two of the case where the expression of the loss of imprinting of the imprinted genes Z10, Z11 and Z13 is less than 15%, the case where the expression of the copy number variation of the imprinted genes Z10, Z11 and Z13 is less than 1.5%, and the case where the total expression of the imprinted genes Z10, Z11 and Z13 is less than 20%;

Grade I: any one or the combination of at least two of the case where the expression of the loss of imprinting of the imprinted genes Z10, Z11 and Z13 is 15-20%, the case where the expression of the copy number variation of the imprinted genes Z10, Z11 and Z13 is 1.5-2.5%, and the case where the total expression of the imprinted genes Z10, Z11 and Z13 is 20-30%;

Grade II: any one or the combination of at least two of the case where the expression of the loss of imprinting of the imprinted genes Z10, Z11 and Z13 is 20-23%, the case where the expression of the copy number variation of the imprinted genes Z10, Z11 and Z13 is 2.5-3.5%, and the case where the total expression of the imprinted genes Z10, Z11 and Z13 is 30-40%;

Grade III: any one or the combination of at least two of the case where the expression of the loss of imprinting of the imprinted genes Z10, Z11 and Z13 is 23-27%, the case where the expression of the copy number variation of the imprinted genes Z10, Z11 and Z13 is 3.5-5%, and the case where the total expression of the imprinted genes Z10, Z11 and Z13 is 40-50%;

Grade IV: any one or the combination of at least two of the case where the expression of the loss of imprinting of the imprinted genes Z10, Z11 and Z13 is greater than 27%, the case where the expression of the copy number variation of the imprinted genes Z10, Z11 and Z13 is greater than 5%, and the case where the total expression of the imprinted genes Z10, Z11 and Z13 is greater than 50%;

The expression of the loss of imprinting, the expression of the copy number variation and the total expression of the imprinted genes Z10, Z11 and Z13 are mutually independent.

In one embodiment, the benign and malignant degree of skin tumors includes benign tumor, skin cancer potential, early skin cancer, intermediate skin cancer and terminal skin cancer.

In one embodiment, if the expression of the loss of imprinting and the expression of the copy number variation of the imprinted genes Z1, Z6, Z8, Z10, Z11, Z13 and Z16 are less than Grade I, or the expression of the loss of imprinting of no more than one of the imprinted gene Z1, Z6, Z8, Z10, Z11, Z13 and Z16 is Grade I, the expression of the copy number variation of no more than one of the Z1, Z6, Z8, Z10, Z11, Z13 and Z16 is Grade I and the expression of the loss of imprinting and the expression of the copy number variation of the imprinted gene Z8 are not both Grade I, the benign and malignant degree of skin tumors is determined as the benign tumor;

If the expression of the loss of imprinting of at least two of the imprinted genes Z1, Z6, Z8, Z10, Z11, Z13 and Z16 is Grade I, the expression of the copy number variation of at least two of the imprinted genes Z1, Z6, Z8, Z10, Z11, Z13 and Z16 is Grade I and the expression of the loss of imprinting and the expression of the copy number variation of the imprinted gene Z8 are both Grade I, or the expression of the loss of imprinting of no more than one of the imprinted genes Z1, Z6, Z8, Z10, Z11, Z13 and Z16 is Grade II and the expression of the copy number variation of no more than one of the imprinted genes Z1, Z6, Z8, Z10, Z11, Z13 and Z16 is Grade II, the benign and malignant degree of skin tumors is determined as the skin cancer potential;

If the expression of the loss of imprinting of at least two of the imprinted genes Z1, Z6, Z8, Z10, Z11, Z13 and Z16 is Grade II and the expression of the copy number variation of at least two of the imprinted genes Z1, Z6, Z8, Z10, Z11, Z13 and Z16 is Grade II, or the expression of the loss of imprinting of no more than one of the imprinted genes Z1, Z6, Z8, Z10, Z11, Z13 and Z16 is Grade III and the expression of the copy number variation of no more than one of the imprinted genes Z1, Z6, Z8, Z10, Z11, Z13 and Z16 is Grade III, the benign and malignant degree of skin tumors is determined as the early skin cancer;

If the expression of the loss of imprinting of at least two of the imprinted genes Z1, Z6, Z8, Z10, Z11, Z13 and Z16 is Grade III and the expression of the copy number variation of at least two of the imprinted genes Z1, Z6, Z8, Z10, Z11, Z13 and Z 16 is Grade III, or the expression of the loss of imprinting of no more than one of the imprinted genes Z1, Z6, Z8, Z10, Z11, Z13 and Z16 is Grade IV and the expression of the copy number variation of no more than one of the imprinted genes Z1, Z6, Z8, Z10, Z11, Z13 and Z16 is Grade IV, the benign and malignant degree of skin tumors is determined as the intermediate skin cancer;

If the expression of the loss of imprinting of at least two of the imprinted genes Z1, Z6, Z8, Z10, Z11, Z13 and Z16 is Grade IV or the expression of the copy number variation of at least two of the imprinted genes Z1, Z6, Z8, Z10, Z11, Z13 and Z16 is Grade IV, the benign and malignant degree of skin tumors is determined as the terminal skin cancer.

In one embodiment, the disclosure further provides an application of the model or the device in detecting skin cancers.

In one embodiment, the disclosure further provides an application of the model or the device in preparing drugs or instruments for treating skin cancers.

In one embodiment, the benign and malignant degree of skin tumors includes benign tumor, skin cancer potential, early skin cancer, intermediate skin cancer and terminal skin cancer;

If the expression of the loss of imprinting and the expression of the copy number variation of the imprinted genes Z1, Z6, Z8, Z10, Z11, Z13 and Z16 are less than Grade I, or the expression of the loss of imprinting of no more than one of the imprinted gene Z1, Z6, Z8, Z10, Z11, Z13 and Z16 is Grade I, the expression of the copy number variation of no more than one of the Z1, Z6, Z8, Z10, Z11, Z13 and Z16 is Grade I and the expression of the loss of imprinting and the expression of the copy number variation of the imprinted gene Z8 are not both Grade I, the benign and malignant degree of skin tumors is determined as the benign tumor;

If the expression of the loss of imprinting of at least two of the imprinted genes Z1, Z6, Z8, Z10, Z11, Z13 and Z16 is Grade I, the expression of the copy number variation of at least two of the imprinted genes Z1, Z6, Z8, Z10, Z11, Z13 and Z16 is Grade I and the expression of the loss of imprinting and the expression of the copy number variation of the imprinted gene Z8 are both Grade I, or the expression of the loss of imprinting of no more than one of the imprinted genes Z1, Z6, Z8, Z10, Z11, Z13 and Z16 is Grade II and the expression of the copy number variation of no more than one of the imprinted genes Z1, Z6, Z8, Z10, Z11, Z13 and Z16 is Grade II, the benign and malignant degree of skin tumors is determined as the skin cancer potential;

If the expression of the loss of imprinting of at least two of the imprinted genes Z1, Z6, Z8, Z10, Z11, Z13 and Z16 is Grade II and the expression of the copy number variation of at least two of the imprinted genes Z1, Z6, Z8, Z10, Z11, Z13 and Z16 is Grade II, or the expression of the loss of imprinting of no more than one of the imprinted genes Z1, Z6, Z8, Z10, Z11, Z13 and Z16 is Grade III and the expression of the copy number variation of no more than one of the imprinted genes Z1, Z6, Z8, Z10, Z11, Z13 and Z16 is Grade III, the benign and malignant degree of skin tumors is determined as the early skin cancer;

If the expression of the loss of imprinting of at least two of the imprinted genes Z1, Z6, Z8, Z10, Z11, Z13 and Z16 is Grade III and the expression of the copy number variation of at least two of the imprinted genes Z1, Z6, Z8, Z10, Z11, Z13 and Z16 is Grade III, or the expression of the loss of imprinting of no more than one of the imprinted genes Z1, Z6, Z8, Z10, Z11, Z13 and Z16 is Grade IV and the expression of the copy number variation of no more than one of the imprinted genes Z1, Z6, Z8, Z10, Z11, Z13 and Z16 is Grade IV, the benign and malignant degree of skin tumors is determined as the intermediate skin cancer;

If the expression of the loss of imprinting of at least two of the imprinted genes Z1, Z6, Z8, Z10, Z11, Z13 and Z16 is Grade IV or the expression of the copy number variation of at least two of the imprinted genes Z1, Z6, Z8, Z10, Z11, Z13 and Z16 is Grade IV, the benign and malignant degree of skin tumors is determined as the terminal skin cancer.

Compared with the prior art, the detection model and device in the embodiments of the specification intuitively express the presentation of imprinted genes on tissue and cell samples of patients with skin tumors, detect the changes of the imprinted genes by means of in-situ marking objectively, intuitively and accurately in an early phase, provide a quantitative model, and make a great contribution to the diagnosis of molecular pathology.

**Brief Description of the Several Views of the Drawings**

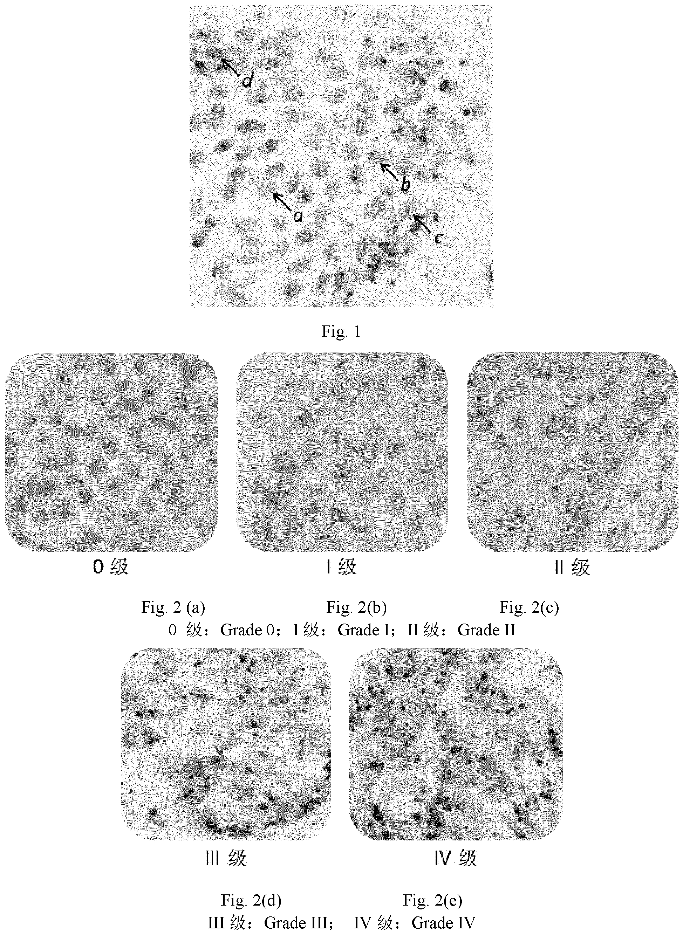

FIG. 1 illustrates a pathological section of a skin cancer after a cell nucleus is stained with hematoxylin, wherein a shows a cell nucleus in which no marker exists and no imprinted gene is expressed after a cell is stained with hematoxylin; b shows a cell nucleus in which one red/brown marker and an imprinted gene exist after a cell is stained with hematoxylin; c shows a cell nucleus in which two red/brown markers exist and subjected to the loss of imprinting after a cell is stained with hematoxylin; and d shows a cell nucleus in which more than two red/brown markers exist and subjected to the copy number variation after a cell is stained with hematoxylin;

FIG. 2(a) illustrates the expression state of seven genes in a pathological section of a grade-0 skin tumor, FIG. 2(b) illustrates the expression state of seven genes in a pathological section of a grade-I skin cancer, FIG. 2(c) illustrates the expression state of seven genes in a pathological section of a grade-II skin cancer, FIG. 2(d) illustrates the expression state of seven genes in a pathological section of a grade-III skin cancer, and FIG. 2(e) illustrates the expression state of seven genes in a pathological section of a grade-IV skin cancer;

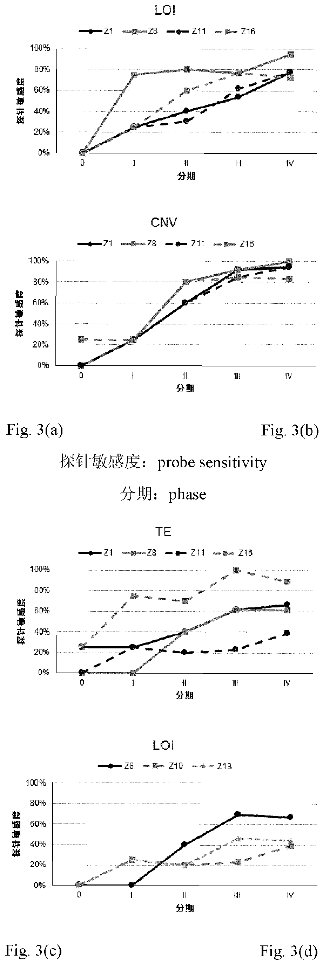

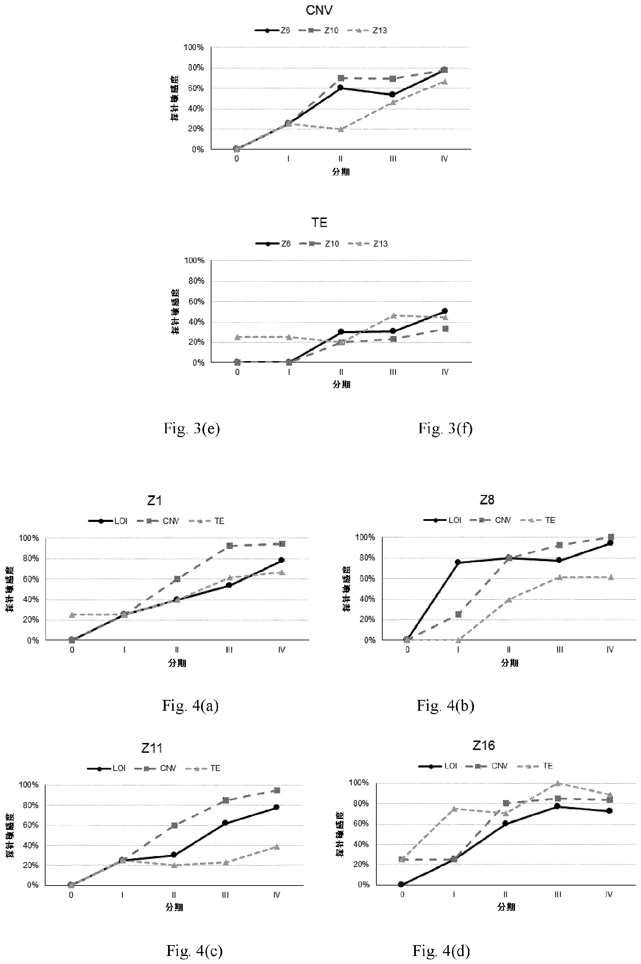

Fig. 3(a) illustrates the degree of the loss of imprinting of imprinted genes Z1, Z8, Z11 and Z16 to skin cancers, Fig. 3(b) illustrates the degree of the copy number variation of the imprinted genes Z1, Z8, Z11 and Z16 to skin cancers, Fig. 3(c) illustrates the degree of the total expression of the imprinted genes imprinted genes Z1, Z8, Z11 and Z16 to skin cancers, Fig. 3(d) illustrates the degree of the loss of imprinting of imprinted genes Z6, Z10 and Z13 to skin cancers, Fig. 3(e) illustrates the degree of the copy number variation of the imprinted genes Z6, Z10 and Z13 to skin cancers, and Fig. 3(f) illustrates the degree of the total expression of the imprinted genes Z6, Z10 and Z13 to skin cancers, wherein the LOI is the gene expression of the loss of imprinting, CNV is the gene expression of the copy number variation, and TE is the total expression of imprinted genes.

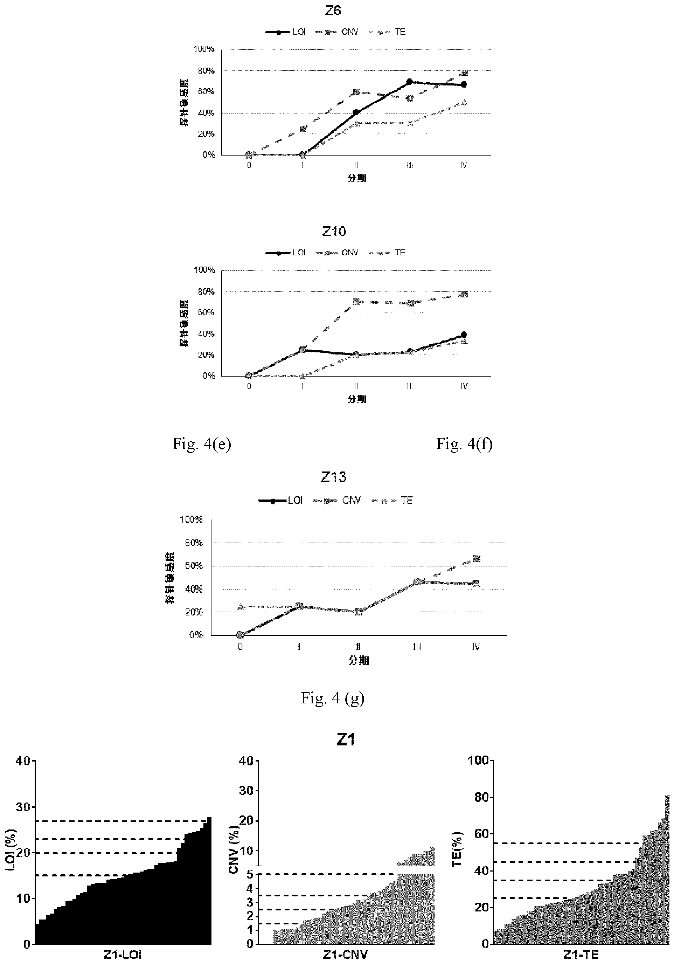

Fig. 4(a) illustrates the degree of the loss of imprinting, copy number variation and total expression of the imprinted gene Z1, Fig. 4(b) illustrates the degree of the loss of imprinting, copy number variation and total expression of the imprinted gene Z8, Fig. 4(c) illustrates the degree of the loss of imprinting, copy number variation and total expression of the imprinted gene Z11, Fig. 4(d) illustrates the degree of the loss of imprinting, copy number variation and total expression of the imprinted gene Z16, Fig. 4(e) illustrates the degree of the loss of imprinting, copy number variation and total expression of the imprinted gene Z6, Fig. 4(f) illustrates the degree of the loss of imprinting, copy number variation and total expression of the imprinted gene Z10, and Fig. 4(g) illustrates the degree of the loss of imprinting, copy number variation and total expression of the imprinted gene Z13, wherein LOI is the gene expression of the loss of imprinting, CNV is the gene expression of the copy number variation, and TE is the total expression of imprinted genes;

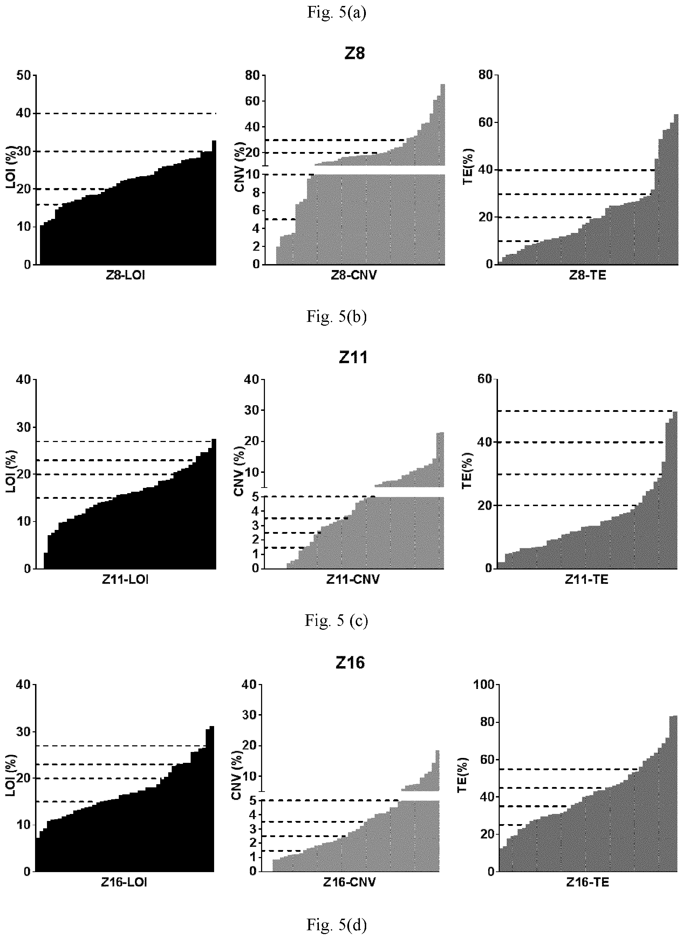

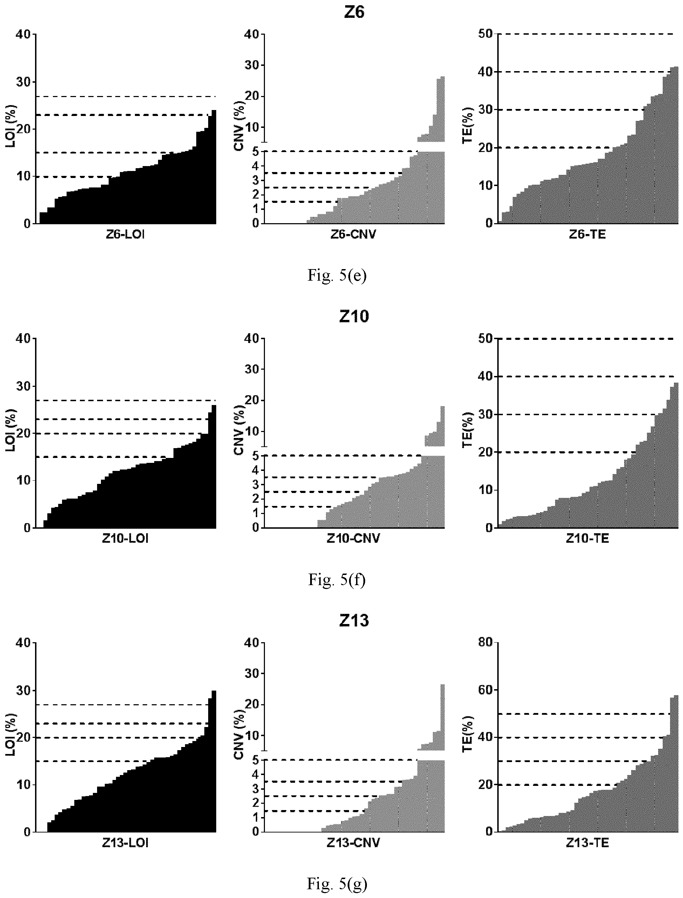

Fig. 5(a) illustrates the distribution range and grading standard of the loss of imprinting, copy number variation and total expression of the imprinted gene Z1 applied to 47 skin cancer pathological sections, Fig. 5(b) illustrates the distribution range and grading standard of the loss of imprinting, copy number variation and total expression of the imprinted gene Z8 applied to 47 skin cancer pathological sections, Fig. 5(c) illustrates the distribution range and grading standard of the loss of imprinting, copy number variation and total expression of the imprinted gene Z11 applied to 47 skin cancer pathological sections, Fig. 5(d) illustrates the distribution range and grading standard of the loss of imprinting, copy number variation and total expression of the imprinted gene Z16 applied to 47 skin cancer pathological sections, Fig. 5(e) illustrates the distribution range and grading standard of the loss of imprinting, copy number variation and total expression of the imprinted gene Z6 applied to 47 skin cancer pathological sections, Fig. 5(f) illustrates the distribution range and grading standard of the loss of imprinting, copy number variation and total expression of the imprinted gene Z10 applied to 47 skin cancer pathological sections, and Fig. 5(g) illustrates the distribution range and grading standard of the loss of imprinting, copy number variation and total expression of the imprinted gene Z13 applied to 47 skin cancer pathological sections, wherein LOI is the gene expression of the loss of imprinting, CNV is the gene expression of the copy number variation, and TE is the total expression of imprinted genes.

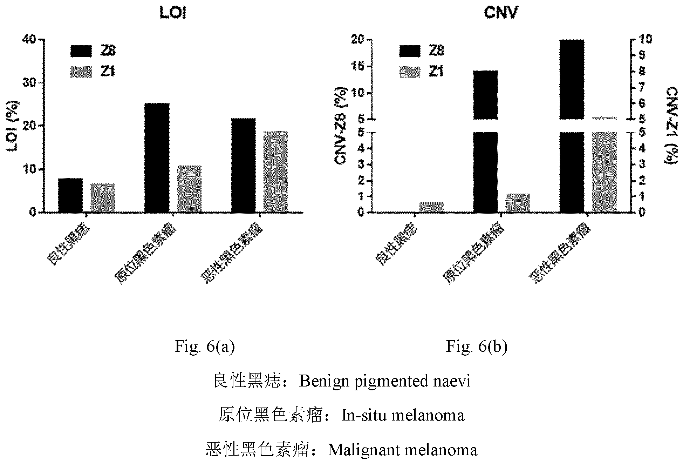

Fig. 6(a) illustrates the expression of the loss of imprinting of the imprinted genes Z1 and Z8 in benign pigmented naevi, in-situ melanoma and malignant melanoma, and Fig. 6(b) illustrates the expression of the copy number variation of the imprinted genes Z1 and Z8 in benign pigmented naevi, in-situ melanoma and malignant melanoma.

## Detailed Description of the Invention

To further expound the technical means and effects of the invention, the technical solutions of the invention will be further explained below in conjunction with the accompanying drawings and specific embodiments. Clearly, the invention is not limited to the scope of the following embodiments.

Genomic imprinting, as a method for gene control in epigenetics, is used to ensure that a certain gene has the expression of only one allele and the other allele is in a gene silencing state by methylating an allele from a specific parental generation. Such a gene is called an imprinted gene. The loss of imprinting is an epigenetic alteration generated when the imprinted gene is demethylated for activating the allele in the silencing state to start gene expression. Many studies indicate that this phenomenon (loss of imprinting) commonly exists in various cancers and occurs before the changes of cells and tissues in form. On the contrary, the proportion of the loss of imprinting in healthy cells is extremely low, which forms a sharp contrast to cancer cells. So, the methylation status of imprinted genes can be used as a pathological marker to analyze the abnormal status of cells through a specific molecular detection technique.

A detection model and system of the disclosure intuitively express the presentation of the loss of imprinting on tissue and cell samples of patients with skin tumors, detect the changes of imprinted genes by means of in-situ marking objectively, intuitively and accurately in an early phase, provide a quantitative model, and make a great contribution to the diagnosis of skin tumors.

A detection device of the disclosure can determine the benign and malignant degree of skin tumors by means of needle biopsy before patients with skin tumors receive a surgery, thus providing a basis of the surgery and accurate treatment, and this is a revolutionary breakthrough of skin tumor diagnosis in the cell molecule field.

This disclosure can accurately determine the type of skin tumors, explicitly grades the benign and malignant degree of skin tumors by means of combined detection of imprinted genes, greatly facilitates the accurate diagnosis of skin cancers in the early phase, is especially suitable for early general investigation, post-operation follow-up of cancers and tracking and follow-up of suspected patients with recurrence, and can save time and make a great contribution to saving patient's life.

The disclosure can accurately distinguish pigmented naevi from in-situ melanoma at the molecular level, can provide a solution to the problem that pigmented naevi and in-situ melanoma cannot be distinguished in tissue and cell morphology at present, and can realize accurate diagnosis of malignant melanoma in the early phase.

Different from the immunohistochemical method, a direction method of the disclosure reduces false positives and other negative effects, and can guide later treatment and drug application through a targeted drug or technical method for silencing, removing, rearranging genes at imprinted gene deletion sites relating to skin tumors.

## Embodiment 1 Analysis of imprinted genes in skin cancers

A detection method for imprinted genes comprises the following steps:

(1) A histocyte section (10$\mu$m) of a skin cancer is acquired and is placed in a 10% neutral formalin solution to be fixed for 24h to prevent RNA degradation and is embedded with parrffin (FFPE), wherein a superfrost plus slide is used, and the section is baked in an oven at 40°C for over 3hrs;
(2) The histocyte section is dewaxed through an RNASCope sample treatment method to block the activity of endogenous Peroxidase in a sample, enhance the permeability and expose RNA molecules;
(3) Probes are designed: specific primers are designed according to an imprinted gene sequence;
Wherein, the probes are designed according to imprinted genes Z1(Gnas), Z6(Plagl1), Z8(Dcn), Z10(Gatm), Z11(Grb10), Z13(Sgce) and Z16(Snrpn/Snurf). Specifically, a fragment is selected in the intron of each gene to be used as a probe. More specifically, the probes are designed by Advanced Cell Diagnostics;
(4) RNASCope in-situ hybridization is carried out on the probes designed in Step (3) and the to-be-detected sample through a kit; and
(5) Signal amplification and hematoxylin staining are carried out, and the expression of imprinted genes is analyzed by means of microscope imaging;

Formulas for calculating the total expression of the imprinted genes, the expression of the loss of imprinting of the imprinted genes, and the expression of the copy number variation of the imprinted genes are as follows:

$$\text{Total expression} = (b+c+d) \,/\, (a+b+c+d) \times 100\%;$$

$$\text{Total expression of normal imprinted genes} = b \,/\, (b+c+d) \times 100\%;$$

$$\text{Expression of the loss of imprinting (LOI)} = c \,/\, (b+c+d) \times 100\%;$$

$$\text{Gene expression of the copy number variation (CNV)} = d \,/\, (b+c+d) \times 100\%;$$

Wherein, as shown in Fig. 1, a is a cell nucleus in which no marker exists and no imprinted gene is expressed after a cell is stained with hematoxylin; b is a cell nucleus in which one red/brown marker and an imprinted gene exist after a cell is stained with hematoxylin; c is a cell nucleus subjected to the loss of imprinting due to the existence two red/brown markers after a cell is stained with hematoxylin; and d is a cell nucleus subjected to the copy number variation due to the existence of more than two red/brown markers after a cell is stained with hematoxylin.

As can be seen from Fig. 2(a) - Fig. 2(e), in the samples from grade 0 to grade IV, the proportion of cells with the loss of imprinting (there are two signal points in the cell nucleus) and copy number variation (there are three or more signal points in the cell nucleus) increases gradually with the increase of the malignant degree.

**Embodiment 2 Analysis of imprinted genes in skin needle biopsy samples**

The skin needle biopsy samples are suspicious pathological tissues taken out by puncture and are blocked in a 10% neutral formalin solution to be fixed for over 24hrs, and other detection steps are the same as those in Embodiment 1.

As can be seen from Fig. 3(a) - Fig. 3(f), the response sensitivities of imprinted genes Z1, Z6, Z8, Z10, Z11, Z13 and Z16 to the skin cancer are different; or, the degrees and statuses of the loss of imprinting for expressing the skin cancer are different.

Specifically, the sensitivity of each imprinted gene to the skin cancer is shown in Fig. 4(a) - Fig. 4(g). As can be seen from Fig. 4(a) - Fig. 4(d), the loss of imprinting of the imprinted gene Z1 appears in a malignant potential phase and gradually rises to a high level with the development of the skin cancer, the copy number variation of the imprinted gene Z1 appears in the malignant potential phase, rises to a high level when the skin cancer develop to an intermediate phase, and maintains in a terminal phase of the skin cancer, and the expression of the imprinted gene Z1 gradually increases to a high level in the early phase and the intermediate phase of the skin cancer and remains stable in the terminal phase of the skin cancer; the loss of imprinting of the imprinted gene Z8 rises rapidly in the malignant potential phase and stops increasing when the skin cancer develops from the early phase to the terminal phase, the copy number variation of the imprinted gene Z8 appears in the malignant potential phase, rapidly rises to a high level in the early phase of the skin cancer and further rises slowly in the intermediate phase and the terminal phase of the skin cancer, and the expression of the imprinted gene Z8 rises rapidly in the early phase and the intermediate phase of the skin cancer and remains stable in the terminal phase of the skin cancer; the loss of imprinting of the imprinted gene Z11 appears in the malignant potential phase, rises slightly in the early phase of the skin cancer, and rises to a high level in the intermediate phase and the terminal phase of the skin cancer, the copy number variation of the imprinted gene Z11 appears in the malignant potential phase and gradually rises to a high level with the development of the skin cancer, and the expression of the imprinted gene Z11 start to rise in the malignant potential phase and does not rise obviously in the development process of the skin cancer; the loss of imprinting and the copy number variation of the imprinted gene Z16 rapidly rise to a high level from the malignant potential phase to the early phase of the skin cancer and maintain in the intermediate phase and the terminal phase of the skin cancer, and the expression of the imprinted gene Z16 rapidly rises to a high level in the malignant potential phase and maintains in the development process of the skin cancer;

As can be seen from Fig. 4(e) - Fig. 4(g), the loss of imprinting of the imprinted gene Z6 rapidly rises to a high level in the early phase and intermediate phase of the skin cancer and remains unchanged in the terminal phase of the skin cancer, the copy number variation of the imprinted gene Z6 appears in the malignant potential phase, rapidly rises to a high level in the early phase of the skin cancer and remains stable in the intermediate phase and the terminal phase of the skin cancer, and the expression of the imprinted gene Z6 starts to rise in the early phase of the skin cancer and does not rise obviously in the intermediate phase and the terminal phase of the skin cancer; the loss of imprinting of the imprinted gene Z10 starts to rise in the malignant potential phase and does not rise obviously from the early phase to the terminal phase of the skin cancer, the copy number variation of the imprinted gene rapidly rises to a high level in the malignant potential phase and the early phase of the skin cancer and further rises in the intermediate phase and the terminal phase of the skin cancer, and the expression of the imprinted gene Z10 starts to rise in the early phase of the skin cancer and does not rise obviously in the intermediate phase and the terminal phase of the skin cancer; the loss of imprinting and the expression of the imprinted gene Z13 do not rise obviously in the malignant potential phase and the early phase of the skin cancer, rises to some extent in the intermediate phase of the skin cancer, and remains stable in the terminal phase of the skin cancer, and the copy number variation of the imprinted gene Z13 does not rise obviously in the malignant potential phase and the early phase of the skin cancer and gradually rises to a high level in the intermediate phase and the terminal phase of the skin cancer.

**Embodiment 3 Analysis of imprinted genes in 47 skin tumor samples**

Tissue and skin needle biopsy samples (10 $\mu$m) of 47 patents with the skin cancer are acquired, and the detection method is the same as that in Embodiment 1.

As can be seen from Fig.5(a) - Fig.5(g), the proportion of the loss of imprinting, the copy number variation and the total expression of seven probes Z1, Z6, Z8, Z10, Z11, Z13 and Z16 is distributed from low to high, the grading standard shown by the dotted line in the figures is obtained by calculation according to the distribution tendency of the probes, and the loss of imprinting, the copy number variation and the total expression of each probe can be classified to five grades from low to high.

The specific grades are as follows:

As can be seen from Fig. 5(a), as for the imprinted gene Z1, any one or the combination of at least two of the case where the expression of the loss of imprinting is less than 15%, the case where the expression of the copy number variation is less than 1.5% and the case where the total expression of the imprinted gene is less than 25% corresponds to grade 0, any one or the combination of at least two of the case where expression of the loss of imprinting is 15-20%, the case where the expression of the copy number variation is 1.5-2.5% and the case where the total expression of the imprinted gene is 25-35% corresponds to grade I, any one or the combination of at least two of the case where expression of the loss of imprinting is 20-23%, the case where the expression of the copy number variation is 2.5-3.5% and the case where the total expression of the imprinted gene is 35-45% corresponds to grade II, any one or the combination of at least two of the case where expression of the loss of imprinting is 23-27%, the case where the expression of the copy number variation is 3.5-5% and the case where the total expression of the imprinted gene is 45-55% corresponds to grade III, and any one or the combination of at least two of the case where expression of the loss of imprinting is greater than 27%, the case where the expression of the copy number variation is greater than 5% and the case where the total expression of the imprinted gene is greater than 55% corresponds to grade IV;

As can be seen from Fig. 5(b), as for the imprinted gene Z8, any one or the combination of at least two of the case where the expression of the loss of imprinting is less than 16%, the case where the expression of the copy number variation is less than 5% and the case where the total expression of the imprinted gene is less than 10% corresponds to grade 0, any one or the combination of at least two of the case where the expression of the loss of imprinting is 16-20%, the case where the expression of the copy number variation is 5-10% and the case where the total expression of the imprinted gene is 10-20% corresponds to grade I, any one or the combination of at least two of the case where the expression of the loss of imprinting is 20-30%, the case where the expression of the copy number variation is 10-20% and the case where the total expression of the imprinted gene is 20-30% corresponds to grade II, any one or the combination of at least two of the case where the expression of the loss of imprinting is 30-40%, the case where the expression of the copy number variation is 20-30% and the case where the total expression of the imprinted gene is 30-40% corresponds to grade III, and any one or the combination of at least two of the case where the expression of the loss of imprinting is greater than 40%, the case where the expression of the copy number variation is greater than 30% and the case where the total expression of the imprinted gene is greater than 40% corresponds to grade IV;

As can be seen from Fig. 5(c), as for the imprinted gene Z11, any one or the combination of at least two of the case where the expression of the loss of imprinting is less than 10%, the case where the expression of the copy number variation is less than 1.5% and the case where the total expression of the imprinted gene is less than 20% corresponds to grade 0, any one or the combination of at least two of the case where the expression of the loss of imprinting is 10-15%, the case where the expression of the copy number variation is 1.5-2.5% and the case where the total expression of the imprinted gene is 20-30% corresponds to grade I, any one or the combination of at least two of the case where the expression of the loss of imprinting is 15-23%, the case where the expression of the copy number variation is 2.5-3.5% and the case where the total expression of the imprinted gene is 30-40% corresponds to grade II, any one or the combination of at least two of the case where the expression of the loss of imprinting is 23-27%, the case where the expression of the copy number variation is 3.5-5% and the case where the total expression of the imprinted gene is 40-50% corresponds to grade III, and any one or the combination of at least two of the case where the expression of the loss of imprinting is greater than 27%, the case where the expression of the copy number variation is greater than 5% and the case where the total expression of the imprinted gene is greater than 50% corresponds to grade IV;

As can be seen from Fig. 5(d), as for the imprinted gene Z16, any one or the combination of at least two of the case where the expression of the loss of imprinting is less than 15%, the case where the expression of the copy number variation is less than 1.5% and the case where the total expression of the imprinted gene is less than 25% corresponds to grade 0, any one or the combination of at least two of the case where the expression of the loss of imprinting is 15-20%, the case where the expression of the copy number variation is 1.5-2.5% and the case where the total expression of the imprinted gene is 25-35% corresponds to grade I, any one or the combination of at least two of the case where the expression of the loss of imprinting is 20-23%, the case where the expression of the copy number variation is 2.5-3.5% and the case where the total expression of the imprinted gene is 35-45% corresponds to grade II, any one or the combination of at least two of the case where the expression of the loss of imprinting is 23-27%, the case where the expression of the copy number variation is 3.5-5% and the case where the total expression of the imprinted gene is 45-55% corresponds to grade III, and any one or the combination of at least two of the case where the expression of the loss of imprinting is greater than 27%, the case where the expression of the copy number variation is greater than 5% and the case where the total expression of the imprinted gene is greater than 55% corresponds to grade IV;

As can be seen from Fig. 5(e), as for the imprinted gene Z6, any one or the combination of at least two of the case where the expression of the loss of imprinting is less than 10%, the case where the expression of the copy number variation is less than 1.5% and the case where the total expression of the imprinted gene is less than 20% corresponds to grade 0, any one or the combination of at least two of the case where the expression of the loss of imprinting is 10-15%, the case where the expression of the copy number variation is 1.5-2.5% and the case where the total expression of the imprinted gene is 20-30% corresponds to grade I, any one or the combination of at least two of the case where the expression of the loss of imprinting is 15-23%, the case where the expression of the copy number variation is 2.5-3.5% and the case where the total expression of the imprinted gene is 30-40% corresponds to grade II, any one or the combination of at least two of the case where the expression of the loss of imprinting is 23-27%, the case where the expression of the copy number variation is 3.5-5% and the case where the total expression of the imprinted gene is 40-50% corresponds to grade III, and any one or the combination of at least two of the case where the expression of the loss of imprinting is greater than 27%, the case where the expression of the copy number variation is greater than 5% and the case where the total expression of the imprinted gene is greater than 50% corresponds to grade IV;

As can be seen from Fig. 5(f), as for the imprinted gene Z10, any one or the combination of at least two of the case where the expression of the loss of imprinting is less than 10%, the case where the expression of the copy number variation is less than 1.5% and the case where the total expression of the imprinted gene is less than 20% corresponds to grade 0, any one or the combination of at least two of the case where the expression of the loss of imprinting is 10-15%, the case where the expression of the copy number variation is 1.5-2.5% and the case where the total expression of the imprinted gene is 20-30% corresponds to grade I, any one or the combination of at least two of the case where the expression of the loss of imprinting is 15-23%, the case where the expression of the copy number variation is 2.5-3.5% and the case where the total expression of the imprinted gene is 30-40% corresponds to grade II, any one or the combination of at least two of the case where the expression of the loss of imprinting is 23-27%, the case where the expression of the copy number variation is 3.5-5% and the case where the total expression of the imprinted gene is 40-50% corresponds to grade III, and any one or the combination of at least two of the case where the expression of the loss of imprinting is greater than 27%, the case where the expression of the copy number variation is greater than 5% and the case where the total expression of the imprinted gene is greater than 50% corresponds to grade IV;

As can be seen from Fig. 5(g), as for the imprinted gene Z13, any one or the combination of at least two of the case where the expression of the loss of imprinting is less than 10%, the case where the expression of the copy number variation is less than 1.5% and the case where the total expression of the imprinted gene is less than 20% corresponds to grade 0, any one or the combination of at least two of the case where the expression of the loss of imprinting is 10-15%, the case where the expression of the copy number variation is 1.5-2.5% and the case where the total expression of the imprinted gene is 20-30% corresponds to grade I, any one or the combination of at least two of the case where the expression of the loss of imprinting is 15-23%, the case where the expression of the copy number variation is 2.5-3.5% and the case where the total expression of the imprinted gene is 30-40% corresponds to grade II, any one or the combination of at least two of the case where the expression of the loss of imprinting is 23-27%, the case where the expression of the copy number variation is 3.5-5% and the case where the total expression of the imprinted gene is 40-50% corresponds to grade III, and any one or the combination of at least two of the case where the expression of the loss of imprinting is greater than 27%, the case where the expression of the copy number variation is greater than 5% and the case where the total expression of the imprinted gene is greater than 50% corresponds to grade IV;

The following conclusion can be drawn by a comprehensive analysis of the 47 skin cancer samples:

The benign and malignant degree of skin tumor includes benign tumor, skin cancer potential, early skin cancer, intermediate skin cancer and terminal skin cancer;

If the expression of the loss of imprinting and the expression of the copy number variation of the imprinted genes Z1, Z6, Z8, Z10, Z11, Z13 and Z16 are less than Grade I, or the expression of the loss of imprinting of no more than one of the imprinted gene Z1, Z6, Z8, Z10, Z11, Z13 and Z16 is Grade I, the expression of the copy number variation of no more than one of the Z1, Z6, Z8, Z10, Z11, Z13 and Z16 is Grade I and the expression of the loss of imprinting and the expression of the copy number variation of the imprinted gene Z8 are not both Grade I, the benign and malignant degree of skin tumors is determined as the benign tumor;

If the expression of the loss of imprinting of at least two of the imprinted genes Z1, Z6, Z8, Z10, Z11, Z13 and Z16 is Grade I, the expression of the copy number variation of at least two of the imprinted genes Z1, Z6, Z8, Z10, Z11, Z13 and Z16 is Grade I and the expression of the loss of imprinting and the expression of the copy number variation of the imprinted gene Z8 are both Grade I, or the expression of the loss of imprinting of no more than one of the imprinted genes Z1, Z6, Z8, Z10, Z11, Z13 and Z16 is Grade II and the expression of the copy number variation of no more than one of the imprinted genes Z1, Z6, Z8, Z10, Z11, Z13 and Z16 is Grade II, the benign and malignant degree of skin tumors is determined as the skin cancer potential;

If the expression of the loss of imprinting of at least two of the imprinted genes Z1, Z6, Z8, Z10, Z11, Z13 and Z16 is Grade II and the expression of the copy number variation of at least two of the imprinted genes Z1, Z6, Z8, Z10, Z11, Z13 and Z16 is Grade II, or the expression of the loss of imprinting of no more than one of the imprinted genes Z1, Z6, Z8, Z10, Z11, Z13 and Z16 is Grade III and the expression of the copy number variation of no more than one of the imprinted genes Z1, Z6, Z8, Z10, Z11, Z13 and Z16 is Grade III, the benign and malignant degree of skin tumors is determined as the early skin cancer;

If the expression of the loss of imprinting of at least two of the imprinted genes Z1, Z6, Z8, Z10, Z11, Z13 and Z16 is Grade III and the expression of the copy number variation of at least two of the imprinted genes Z1, Z6, Z8, Z10, Z11, Z13 and Z16 is Grade III, or the expression of the loss of imprinting of no more than one of the imprinted genes Z1, Z6, Z8, Z10, Z11, Z13 and Z16 is Grade IV and the expression of the copy number variation of no more than one of the imprinted genes Z1, Z6, Z8, Z10, Z11, Z13 and Z16 is Grade IV, the benign and malignant degree of skin tumors is determined as the intermediate skin cancer;

If the expression of the loss of imprinting of at least two of the imprinted genes Z1, Z6, Z8, Z10, Z11, Z13 and Z16 is Grade IV or the expression of the copy number variation of at least two of the imprinted genes Z1, Z6, Z8, Z10, Z11, Z13 and Z16 is Grade IV, the benign and malignant degree of skin tumors is determined as the terminal skin cancer.

**Embodiment 4 Analysis of imprinted genes in 18 pigmented naevi samples and 18 melanoma samples**

Tissues including skin needle biopsy samples (10μm) of 18 patients with pigmented naevi and 18 patients with melanoma are acquired, and the detection method is the same as that in Embodiment 1.

As can be seen from Fig. 6(a) - Fig. 6(b), the loss of imprinting and the copy number variation of the imprinted genes Z1 and Z8 in benign pigmented naevi are both at a low level, the loss of imprinting and the copy number variation of the imprinted gene Z1 do not increase obviously, but the loss of imprinting and the copy number variation of the imprinted gene Z8 increase drastically. The loss of imprinting and the copy number variation of the imprinted genes Z1 and Z8 in the malignant melanoma both reach a high level. Thus, the imprinted gene Z8 can more sensitively detect the in-situ melanoma in an early phase.

To sum up, the detection model and system intuitively express the presentation of the loss of imprinting on samples of patients with skin tumors, detect the changes of imprinted genes by means of in-situ marking objectively, intuitively and accurately in an early phase, provide a quantitative model, and make a great contribution to the diagnosis of skin tumors.

The applicant states that the detailed methods of the invention have been explained with reference to the above embodiments, but the invention is not limited to these detailed methods, which means that the invention can also be implemented independent of these detailed methods. Those skilled in the art would appreciate that any improvements on this invention, equivalent substitutions of raw materials of the products of the invention and the addition of auxiliary elements, and the selection of specific methods should also fall within the protection scope of the invention and the scope of the disclosure.

**Claims**

1. A grading model for detecting the benign and malignant degree of skin tumors, grading expression states of imprinted genes by calculating changes of an expression of the loss of imprinting of the imprinted genes, an expression of the copy number variation of the imprinted genes and a total expression of the imprinted genes in skin tumors;

wherein, the imprinted genes are any one or the combination of at least two of Z1, Z8, Z11 and Z16, the imprinted gene Z1 is Gnas, the imprinted gene Z8 is Dcn, the imprinted gene Z11 is Grb10, and the imprinted gene Z16 is Snrpn/Snurf.

2. The model according to Claim 1, wherein an imprinted gene calculation method of the model comprises: calculating any one of Z1, Z8, Z11 and Z16, preferably any one of Z1, Z8 and Z11, and further preferably any one of Z1 and Z8.

3. The model according to Claim 1 or 2, wherein the imprinted gene calculation method of the model comprises: calculating the combination of any two of the imprinted genes Z1, Z8, Z11 and Z16, and preferably the combination of Z1 and Z8 or the combination of Z8 and Z11.

4. The model according to any one of Claims 1-3, wherein the imprinted genes further include any one or the combination of at least two of Z6, Z10 and Z13, wherein the imprinted gene Z6 is Plagl1, the imprinted gene Z10 is Gatm, and the imprinted gene Z13 is Sgce.

5. The model according to any one of Claims 1-4, wherein the imprinted gene calculation method of the model comprises: calculating the combination of the seven imprinted genes Z1, Z6, Z8, Z10, Z11, Z13 and Z16.

6. The model according to any one of Claims 1-5, wherein formulas for calculating the total expression of the imprinted genes, the expression of the loss of imprinting of the imprinted genes, and the expression of the copy number variation of the imprinted genes are as follows:

$$\text{total expression} = (b+c+d) \,/\, (a+b+c+d) \times 100\%;$$

$$\text{total expression of normal imprinted genes} = b \,/\, (b+c+d) \times 100\%;$$

$$\text{expression of the loss of imprinting (LOI)} = c \,/\, (b+c+d) \times 100\%;$$

$$\text{gene expression of the copy number variation of imprinted genes (CNV)} = d \,/\, (b+c+d) \times 100\%;$$

wherein, a is a cell nucleus in which no marker exists and no imprinted gene is expressed after a cell is stained with hematoxylin; b is a cell nucleus in which one red/brown marker and an imprinted gene exist after a cell is stained with hematoxylin; c is a cell nucleus in which two red/brown markers exist and subjected to the loss of imprinting after a cell is stained with hematoxylin; and d is a cell nucleus in which more than two red/brown markers exist and subjected to the copy number variation after a cell is stained with hematoxylin.

7. The model according to any one of Claims 1-6, wherein the expression of the loss of imprinting of the imprinted genes, the expression of the copy number variation of the imprinted genes and the total expression of the imprinted genes are classified to five grades.

8. The model according to Claim 7, wherein the expression of the loss of imprinting, the expression of the copy number variation and the total expression of the seven imprinted genes Z1, Z6, Z8, Z10, Z11, Z13 and Z16 are classified to five grades;
the five grades of the expression of the loss of imprinting, the expression of the copy number variation and the total expression of the imprinted genes Z1 and Z16 are:

Grade 0: any one or the combination of at least two of the case where the expression of the loss of imprinting of the imprinted genes Z1 and Z16 is less than 15%, the case where the expression of the copy number variation of the imprinted genes Z1 and Z16 is less than 1.5%, and the case where the total expression of the imprinted genes Z1 and Z16 is less than 25%;
Grade I: any one or the combination of at least two of the case where the expression of the loss of imprinting of the imprinted genes Z1 and Z16 is 15-20%, the case where the expression of the copy number variation of the imprinted genes Z1 and Z16 is 1.5-2.5%, and the case where the total expression of the imprinted genes Z1 and Z16 is 25-35%;

Grade II: any one or the combination of at least two of the case where the expression of the loss of imprinting of the imprinted genes Z1 and Z16 is 20-23%, the case where the expression of the copy number variation of the imprinted genes Z1 and Z16 is 2.5-3.5%, and the case where the total expression of the imprinted genes Z1 and Z16 is 35-45%;

Grade III: any one or the combination of at least two of the case where the expression of the loss of imprinting of the imprinted genes Z1 and Z16 is 23-27%, the case where the expression of the copy number variation of the imprinted genes Z1 and Z16 is 3.5-5%, and the case where the total expression of the imprinted genes Z1 and Z16 is 45-55%;

Grade IV: any one or the combination of at least two of the case where the expression of the loss of imprinting of the imprinted genes Z1 and Z16 is greater than 27%, the case where the expression of the copy number variation of the imprinted genes Z1 and Z16 is greater than 5%, and the case where the total expression of the imprinted genes Z1 and Z16 is greater than 55%;

the five grades of the expression of the loss of imprinting, the expression of the copy number variation and the total expression of the imprinted gene Z6 are:

Grade 0: any one or the combination of at least two of the case where the expression of the loss of imprinting of the imprinted gene Z6 is less than 10%, the case where the expression of the copy number variation of the imprinted gene Z6 is less than 1.5%, and the case where the total expression of the imprinted gene Z6 is less than 20%;

Grade I: any one or the combination of at least two of the case where the expression of the loss of imprinting of the imprinted gene Z6 is 10-15%, the case where the expression of the copy number variation of the imprinted gene Z6 is 1.5-2.5%, and the case where the total expression of the imprinted gene Z6 is 20-30%;

Grade II: any one or the combination of at least two of the case where the expression of the loss of imprinting of the imprinted gene Z6 is 15-23%, the case where the expression of the copy number variation of the imprinted gene Z6 is 2.5-3.5%, and the case where the total expression of the imprinted gene Z6 is 30-40%;

Grade III: any one or the combination of at least two of the case where the expression of the loss of imprinting of the imprinted gene Z6 is 23-27%, the case where the expression of the copy number variation of the imprinted gene Z6 is 3.5-5%, and the case where the total expression of the imprinted gene Z6 is 40-50%;

Grade IV: any one or the combination of at least two of the case where the expression of the loss of imprinting of the imprinted gene Z6 is greater than 27%, the case where the expression of the copy number variation of the imprinted gene Z6 is greater than 5%, and the case where the total expression of the imprinted gene Z6 is greater than 50%.

the five grades of the expression of the loss of imprinting, the expression of the copy number variation and the total expression of the imprinted gene Z8 are:

Grade 0: any one or the combination of at least two of the case where the expression of the loss of imprinting of the imprinted gene Z8 is less than 16%, the case where the expression of the copy number variation of the imprinted gene Z8 is less than 5%, and the case where the total expression of the imprinted gene Z8 is less than 10%;

Grade I: any one or the combination of at least two of the case where the expression of the loss of imprinting of the imprinted gene Z8 is 16-20%, the case where the expression of the copy number variation of the imprinted gene Z8 is 5-10%, and the case where the total expression of the imprinted gene Z8 is 10-20%;

Grade II: any one or the combination of at least two of the case where the expression of the loss of imprinting of the imprinted gene Z8 is 20-30%, the case where the expression of the copy number variation of the imprinted gene Z8 is 10-20%, and the case where the total expression of the imprinted gene Z8 is 20-30%;

Grade III: any one or the combination of at least two of the case where the expression of the loss of imprinting of the imprinted gene Z8 is 30-40%, the case where the expression of the copy number variation of the imprinted gene Z8 is 20-30%, and the case where the total expression of the imprinted gene Z8 is 30-40%;

Grade IV: any one or the combination of at least two of the case where the expression of the loss of imprinting of the imprinted gene Z8 is greater than 40%, the case where the expression of the copy number variation of the imprinted gene Z8 is greater than 30%, and the case where the total expression of the imprinted gene Z8 is greater than 40%;

the five grades of the expression of the loss of imprinting, the expression of the copy number variation and the total expression of the imprinted genes Z10, Z11 and Z 13 are:

Grade 0: any one or the combination of at least two of the case where the expression of the loss of imprinting of the imprinted genes Z10, Z11 and Z13 is less than 15%, the case where the expression of the copy number variation of the imprinted genes Z10, Z11 and Z13 is less than 1.5%, and the case where the total expression of the imprinted genes Z10, Z11 and Z13 is less than 20%;

Grade I: any one or the combination of at least two of the case where the expression of the loss of imprinting of the imprinted genes Z10, Z11 and Z13 is 15-20%, the case where the expression of the copy number variation of the imprinted genes Z10, Z11 and Z13 is 1.5-2.5%, and the case where the total expression of the imprinted genes Z10, Z11 and Z13 is 20-30%;

Grade II: any one or the combination of at least two of the case where the expression of the loss of imprinting of the imprinted genes Z10, Z11 and Z13 is 20-23%, the case where the expression of the copy number variation of the imprinted genes Z10, Z11 and Z13 is 2.5-3.5%, and the case where the total expression of the imprinted genes Z10, Z11 and Z13 is 30-40%;

Grade III: any one or the combination of at least two of the case where the expression of the loss of imprinting of the imprinted genes Z10, Z11 and Z13 is 23-27%, the case where the expression of the copy number variation of the imprinted genes Z10, Z11 and Z13 is 3.5-5%, and the case where the total expression of the imprinted genes Z10, Z11 and Z13 is 40-50%;

Grade IV: any one or the combination of at least two of the case where the expression of the loss of imprinting of the imprinted genes Z10, Z11 and Z13 is greater than 27%, the case where the expression of the copy number variation of the imprinted genes Z10, Z11 and Z13 is greater than 5%, and the case where the total expression of the imprinted genes Z10, Z11 and Z13 is greater than 50%;

9. A device for detecting the benign and malignant degree of skin tumors, adopting the model according to any one of Claims 1-8, and comprising:

(1) a sampling unit for acquiring to-be-detected samples;
(2) a probe design unit for designing specific primers according to an imprinted gene sequence;
(3) a detection unit for carrying out in-situ hybridization on probes designed in Step (2) and the to-be-detected samples; and
(4) an analysis unit for analyzing expressions of imprinted genes by means of microscope imaging;

wherein, the analysis unit calculates an expression of the loss of imprinting, an expression of the copy number variation and a total expression of imprinted genes, and then, the benign and malignant degree of skin tumors is determined through the model according to any one of Claims 1-8 according to the grade of the expression of the loss of imprinting, the expression of the copy number variation and the total expression of the imprinted genes.

10. A method for detecting the benign and malignant degree of skin tumors, adopting the model according to any one of Claims 1-8, or the device according to Claim 9, and comprising:

(1) acquiring to-be-detected samples;
(2) designing specific primers according to an imprinted gene sequence;
(3) carrying out in-situ hybridization on probes designed in Step (2) and the to-be-detected samples; and
(4) analyzing expressions of imprinted genes by means of microscope imaging to diagnose the benign and malignant degree of skin tumors;

wherein, the analysis unit calculates the expression of the loss of imprinting, the expression of the copy number variation and the total expression of the imprinted genes, and then, the benign and malignant degree of skin tumors is determined through the model according to any one of Claims 1-8 according to the grade of the expression of the loss of imprinting, the expression of the copy number variation and the total expression of the imprinted genes.

11. The method according to Claim 10, wherein the to-be-tested samples acquired in Step (1) are tissues and/or cells of humans.

12. The method according to Claim 10 or 11, wherein to-be-tested samples are paraffin sections of skin and/or needle biopsy samples.

13. The method according to any one of Claims 10-12, wherein the in-situ hybridization is RNAscope in-situ hybridization.

14. The method according to any one of Claims 10-13, wherein the RNAscope in-situ hybridization uses a single-channel or multi-channel chromogenic kit, or a single-channel or multi-channel fluorescent kit, and is preferably a single-channel red/brown chromogenic kit or a multi-channel fluorescent kit.

15. The method according to any one of Claims 10-14, wherein the benign and malignant degree of skin tumors includes benign tumor, skin cancer potential, early skin cancer, intermediate skin cancer and terminal skin cancer.

16. The method according to any one of Claims 10-15, wherein if the expression of the loss of imprinting and the expression of the copy number variation of the imprinted genes Z1, Z6, Z8, Z10, Z11, Z13 and Z16 are less than Grade I, or the expression of the loss of imprinting of no more than one of the imprinted gene Z1, Z6, Z8, Z10, Z11, Z13 and Z16 is Grade I, the expression of the copy number variation of no more than one of the Z1, Z6, Z8, Z10, Z11, Z13 and Z16 is Grade I and the expression of the loss of imprinting and the expression of the copy number variation of the imprinted gene Z8 are not both Grade I, the benign and malignant degree of skin tumors is determined as the benign tumor;
if the expression of the loss of imprinting of at least two of the imprinted genes Z1, Z6, Z8, Z10, Z11, Z13 and Z 16 is Grade I, the expression of the copy number variation of at least two of the imprinted genes Z1, Z6, Z8, Z10, Z11, Z13 and Z16 is Grade I and the expression of the loss of imprinting and the expression of the copy number variation of the imprinted gene Z8 are both Grade I, or the expression of the loss of imprinting of no more than one of the imprinted genes Z1, Z6, Z8, Z10, Z11, Z13 and Z16 is Grade II and the expression of the copy number variation of no more than one of the imprinted genes Z1, Z6, Z8, Z10, Z11, Z13 and Z16 is Grade II, the benign and malignant degree of skin tumors is determined as the skin cancer potential;
if the expression of the loss of imprinting of at least two of the imprinted genes Z1, Z6, Z8, Z10, Z11, Z13 and Z16 is Grade II and the expression of the copy number variation of at least two of the imprinted genes Z1, Z6, Z8, Z10, Z11, Z13 and Z16 is Grade II, or the expression of the loss of imprinting of no more than one of the imprinted genes Z1, Z6, Z8, Z10, Z11, Z13 and Z16 is Grade III and the expression of the copy number variation of no more than one of the imprinted genes Z1, Z6, Z8, Z10, Z11, Z13 and Z16 is Grade III, the benign and malignant degree of skin tumors is determined as the early skin cancer;
if the expression of the loss of imprinting of at least two of the imprinted genes Z1, Z6, Z8, Z10, Z11, Z13 and Z16 is Grade III and the expression of the copy number variation of at least two of the imprinted genes Z1, Z6, Z8, Z10, Z11, Z13 and Z16 is Grade III, or the expression of the loss of imprinting of no more than one of the imprinted genes Z1, Z6, Z8, Z10, Z11, Z13 and Z16 is Grade IV and the expression of the copy number variation of no more than one of the imprinted genes Z1, Z6, Z8, Z10, Z11, Z13 and Z16 is Grade IV, the benign and malignant degree of skin tumors is determined as the intermediate skin cancer;
if the expression of the loss of imprinting of at least two of the imprinted genes Z1, Z6, Z8, Z10, Z11, Z13 and Z16 is Grade IV or the expression of the copy number variation of at least two of the imprinted genes Z1, Z6, Z8, Z10, Z11, Z13 and Z16 is Grade IV, the benign and malignant degree of skin tumors is determined as the terminal skin cancer.

17. An application of the model according to any one of Claims 1-8 or the device according to Claim 9 in detecting skin cancers.

18. An application of the model according to any one of Claims 1-8 or the device according to Claim 9 in preparing drugs or instruments for treating skin cancers.

19. The application according to Claim 17 or 18, wherein the benign and malignant degree of skin tumors includes benign tumor, skin cancer potential, early skin cancer, intermediate skin cancer and terminal skin cancer.

20. The application according to any one of Claims 17-19, wherein if the expression of the loss of imprinting and the expression of the copy number variation of the imprinted genes Z1, Z6, Z8, Z10, Z11, Z13 and Z16 are less than Grade I, or the expression of the loss of imprinting of no more than one of the imprinted gene Z1, Z6, Z8, Z10, Z11, Z13 and Z16 is Grade I, the expression of the copy number variation of no more than one of the Z1, Z6, Z8, Z10, Z11, Z13 and Z16 is Grade I and the expression of the loss of imprinting and the expression of the copy number variation of the imprinted gene Z8 are not both Grade I, the benign and malignant degree of skin tumors is determined as the benign tumor;
if the expression of the loss of imprinting of at least two of the imprinted genes Z1, Z6, Z8, Z10, Z11, Z13 and Z16 is Grade I, the expression of the copy number variation of at least two of the imprinted genes Z1, Z6, Z8, Z10, Z11, Z13 and Z16 is Grade I and the expression of the loss of imprinting and the expression of the copy number variation of the imprinted gene Z8 are both Grade I, or the expression of the loss of imprinting of no more than one of the imprinted genes Z1, Z6, Z8, Z10, Z11, Z13 and Z16 is Grade II and the expression of the copy number variation of no more than one of the imprinted genes Z1, Z6, Z8, Z10, Z11, Z13 and Z16 is Grade II, the benign and malignant degree of skin tumors is determined as the skin cancer potential;

if the expression of the loss of imprinting of at least two of the imprinted genes Z1, Z6, Z8, Z10, Z11, Z13 and Z16 is Grade II and the expression of the copy number variation of at least two of the imprinted genes Z1, Z6, Z8, Z10, Z11, Z13 and Z16 is Grade II, or the expression of the loss of imprinting of no more than one of the imprinted genes Z1, Z6, Z8, Z10, Z11, Z13 and Z16 is Grade III and the expression of the copy number variation of no more than one of the imprinted genes Z1, Z6, Z8, Z10, Z11, Z13 and Z16 is Grade III, the benign and malignant degree of skin tumors is determined as the early skin cancer;

if the expression of the loss of imprinting of at least two of the imprinted genes Z1, Z6, Z8, Z10, Z11, Z13 and Z16 is Grade III and the expression of the copy number variation of at least two of the imprinted genes Z1, Z6, Z8, Z10, Z11, Z13 and Z16 is Grade III, or the expression of the loss of imprinting of no more than one of the imprinted genes Z1, Z6, Z8, Z10, Z11, Z13 and Z16 is Grade IV and the expression of the copy number variation of no more than one of the imprinted genes Z1, Z6, Z8, Z10, Z11, Z13 and Z16 is Grade IV, the benign and malignant degree of skin tumors is determined as the intermediate skin cancer;

if the expression of the loss of imprinting of at least two of the imprinted genes Z1, Z6, Z8, Z10, Z11, Z13 and Z16 is Grade IV or the expression of the copy number variation of at least two of the imprinted genes Z1, Z6, Z8, Z10, Z11, Z13 and Z16 is Grade IV, the benign and malignant degree of skin tumors is determined as the terminal skin cancer.

Fig. 1

0 级        I 级        II 级

Fig. 2 (a)        Fig. 2(b)        Fig. 2(c)

0 级：Grade 0；I 级：Grade I；II 级：Grade II

III 级        IV 级

Fig. 2(d)        Fig. 2(e)

III 级：Grade III； IV 级：Grade IV

## LOI

## CNV

Fig. 3(a)                                        Fig. 3(b)

探针敏感度：probe sensitivity

分期：phase

## TE

## LOI

Fig. 3(c)                                        Fig. 3(d)

CNV

TE

Fig. 3(e)                                    Fig. 3(f)

Z1

Z8

Fig. 4(a)                                    Fig. 4(b)

Z11

Z16

Fig. 4(c)                                    Fig. 4(d)

Z6

Z10

Fig. 4(e)                                              Fig. 4(f)

Z13

Fig. 4 (g)

Z1

Fig. 5(a)

**Z8**

Fig. 5(b)

**Z11**

Fig. 5 (c)

**Z16**

Fig. 5(d)

Fig. 5(e)

Fig. 5(f)

Fig. 5(g)

Fig. 6(a)                                   Fig. 6(b)

良性黑痣：Benign pigmented naevi

原位黑色素瘤：In-situ melanoma

恶性黑色素瘤：Malignant melanoma

## INTERNATIONAL SEARCH REPORT

| International application No. |
|---|
| **PCT/CN2019/104946** |

**A.  CLASSIFICATION OF SUBJECT MATTER**

C12Q 1/6888(2018.01)i;  C12Q 1/6886(2018.01)i

According to International Patent Classification (IPC) or to both national classification and IPC

**B.  FIELDS SEARCHED**

Minimum documentation searched (classification system followed by classification symbols)

C12Q

Documentation searched other than minimum documentation to the extent that such documents are included in the fields searched

Electronic data base consulted during the international search (name of data base and, where practicable, search terms used)

CNABS, DWPI, CNKI, 万方数据, WANFANG DATA, ISI Web of Knowledge, Google Scholar, 百度学术, BAIDU SCHOLAR, Patentics: 分级, 癌, 恶性, 印记, snrpn, snurf, 黑色素瘤, 基底细胞瘤, gnas, dcn, grb10, 标记, 肿瘤, 模型, 基因, plag11, 良恶性, sgce, dcn, gatm, melanoma, skin cancer, basal cell, squamous cell, model, grading, grade

**C.  DOCUMENTS CONSIDERED TO BE RELEVANT**

| Category* | Citation of document, with indication, where appropriate, of the relevant passages | Relevant to claim No. |
|---|---|---|
| Y | WO 9524503 A1 (RAPAPORT, E. et al.) 14 September 1995 (1995-09-14) claims 1-10 | 1-20 |
| Y | CN 102421920 A (NATIONAL RESEARCH COUNCIL CANADA) 18 April 2012 (2012-04-18) claims 1-28, and embodiment 1 | 1-20 |
| Y | ONKEN, M. D. et al. "Oncogenic Mutations in GNAQ Occur Early in Uveal Melanoma" *INVEST OPHTHALMOL VIS SCI*, Vol. 12, No. 49, 31 December 2008 (2008-12-31), pp. 5230-5234 | 1-20 |
| Y | ZHANG, W. et al. "Decorin is a pivotal effector in the extracellular matrix and tumour microenvironment" *ONCOTARGET*, Vol. 4, No. 9, 03 January 2018 (2018-01-03), pp. 5480-5491 | 1-20 |
| Y | DARR, O. A. et al. "Epigenetic alterations in metastatic cutaneous carcinoma" *HEAD & NECK.*, Vol. 37, No. 7, 27 June 2014 (2014-06-27), pp. 994-1001 | 1-20 |

☑ Further documents are listed in the continuation of Box C.   ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **27 November 2019** | **05 December 2019** |

| Name and mailing address of the ISA/CN | Authorized officer |
|---|---|
| **China National Intellectual Property Administration (ISA/ CN)** **No. 6, Xitucheng Road, Jimenqiao Haidian District, Beijing 100088 China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2019/104946** |

**C.    DOCUMENTS CONSIDERED TO BE RELEVANT**

| Category* | Citation of document, with indication, where appropriate, of the relevant passages | Relevant to claim No. |
| --- | --- | --- |
| Y | MA, J. et al. "Small nuclear ribonucleoprotein associated polypeptide N accelerates cell proliferation in pancreatic adenocarcinoma" *Molecular Medicine Reports*, Vol. 12, 31 December 2015 (2015-12-31), pp. 6060-6064 | 1-20 |
| Y | BASYUK, E. et al. "The Candidate Tumor Suppressor Gene ZAC Is Involved in Keratinocyte Differentiation and Its Expression Is Lost in Basal Cell Carcinomas" *MOLECULAR CANCER RESEARCH,* Vol. 3, No. 9, 30 September 2005 (2005-09-30), pp. 483-492 | 1-20 |
| Y | DONG, H. et al. "Digital karyotyping reveals probable target genes at 7q21.3 locus in hepatocellular carcinoma" *BMC MEDICAL GENOMICS*, Vol. 4, 19 July 2011 (2011-07-19), Artical no.60 | 1-20 |

Form PCT/ISA/210 (second sheet) (January 2015)

## INTERNATIONAL SEARCH REPORT
### Information on patent family members

International application No.

**PCT/CN2019/104946**

| Patent document cited in search report | | | Publication date (day/month/year) | Patent family member(s) | | | Publication date (day/month/year) |
|---|---|---|---|---|---|---|---|
| WO | 9524503 | A1 | 14 September 1995 | EP | 0759092 | A1 | 26 February 1997 |
| | | | | DE | 69526855 | T2 | 28 November 2002 |
| | | | | IL | 108879 | D0 | 24 June 1994 |
| | | | | EP | 0759092 | B1 | 29 May 2002 |
| | | | | US | 5955273 | A | 21 September 1999 |
| | | | | DE | 69526855 | D1 | 04 July 2002 |
| | | | | AU | 1893095 | A | 25 September 1995 |
| | | | | ES | 2177653 | T3 | 16 December 2002 |
| | | | | IL | 108879 | A | 31 August 2000 |
| CN | 102421920 | A | 18 April 2012 | CA | 2758041 | A1 | 21 October 2010 |
| | | | | CN | 105132544 | A | 09 December 2015 |
| | | | | EP | 2419533 | A1 | 22 February 2012 |
| | | | | CN | 105200124 | A | 30 December 2015 |
| | | | | JP | 2012525818 | A | 25 October 2012 |
| | | | | US | 2012040863 | A1 | 16 February 2012 |
| | | | | EP | 2419533 | A4 | 31 December 2014 |
| | | | | WO | 2010118520 | A1 | 21 October 2010 |
| | | | | JP | 2016073287 | A | 12 May 2016 |
| | | | | AU | 2010237568 | A1 | 17 November 2011 |
| | | | | CN | 102421920 | B | 30 September 2015 |

Form PCT/ISA/210 (patent family annex) (January 2015)